# EUROPEAN PATENT APPLICATION

(11) **EP 3 438 101 A1**
(43) Date of publication of application: **06.02.2019**
(21) Application number: 17184182.8
(22) Date of filing: 01.08.2017
(51) Int. Cl.: C07D 301/12

(54) **PROCESS FOR THE EPOXIDATION OF PROPENE**

(71) Applicant: Evonik Degussa GmbH, 45128 Essen (DE); thyssenkrupp Industrial Solutions AG, 45143 Essen (DE)
(72) Inventor: MORELL, Heiko, 63110 Rodgau (DE); WIELAND, Stefan, 63456 Hanau (DE); BERNHARD, Andrea, 60388 Frankfurt (DE); GAUDSCHUN, Kurt-Alfred, 45749 Oer-Erkenschwick (DE); PASCALY, Matthias, 60599 Frankfurt (DE); WÖLL, Wolfgang, 63477 Maintal (DE); SCHMIDT, Franz, 60389 Frankfurt (DE)

(57) **Abstract**

In a process for the epoxidation of propene with hydrogen peroxide in the presence of an epoxidation catalyst, an epoxidation catalyst is used comprising a first titanium silicalite with a titanium content of from 1.0 to 3.0 % by weight, calculated as TiO₂, and a second titanium silicalite with a titanium content of from 3.0 to 5.0 % by weight, the titanium content of the second titanium silicalite being at least 0.1 % by weight higher than the titanium content of the first titanium silicalite.

## Description

### Field of the invention

The present invention relates to a process for the epoxidation of propene with hydrogen peroxide in the presence of an epoxidation catalyst comprising a titanium silicalite.

### Background of the invention

The epoxidation of propene with hydrogen peroxide in the presence of a titanium silicalite catalyst is known from EP 0 100 119 A1.

WO 2004/098769 describes reactions of organic compounds with hydroperoxides in the presence of two different zeolite catalysts which are spatially separated from each other. The examples disclose an epoxidation of propene with hydrogen peroxide in a tube bundle reactor using two catalysts shaped as extrudates, containing titanium silicalites with different titanium contents, arranged in different sections of the tubes.

### Summary of the invention

It has now surprisingly been found that an epoxidation of propene with an epoxidation catalyst containing a mixture of two titanium silicalites which differ in their titanium content provides better yields of propene oxide than the use of a single titanium silicalite having on average the same titanium content or the use of the same titanium silicalites in spatially separated catalysts.

Subject of the invention is therefore a process for the epoxidation of propene with hydrogen peroxide in the presence of an epoxidation catalyst, wherein the epoxidation catalyst comprises a first titanium silicalite with a titanium content of from 1.0 to 3.0 % by weight, calculated as TiO₂, and a second titanium silicalite with a titanium content of from 3.0 to 5.0 % by weight, calculated as TiO₂, the titanium content of the second titanium silicalite being at least 0.1 % by weight, calculated as TiO₂, higher than the titanium content of the first titanium silicalite.

### Detailed description of the invention

In the process of the invention, propene is reacted with hydrogen peroxide in the presence of an epoxidation catalyst comprising a titanium silicalite.

The propene used in the process may contain propane, preferably with a molar ratio of propane to propene of from 0.001 to 0.15 and more preferably of from 0.08 to 0.12. Hydrogen peroxide can be used as an aqueous solution, preferably containing from 30 to 75 % by weight hydrogen peroxide and most preferably from 40 to 70 % by weight. Preferably, an aqueous hydrogen peroxide solution made by an anthraquinone process is used. The molar ratio of propene to hydrogen peroxide is preferably from 1.1:1 to 30:1, more preferably 2:1 to 10:1 and most preferably 3:1 to 5:1.

The epoxidation is carried out in the presence of an epoxidation catalyst comprising a titanium silicalite, preferably a titanium silicalite with an MFI or MEL crystal structure. Most preferably a titanium silicalite 1 with MFI structure as known from EP 0 100 119 A1, is used. The epoxidation catalyst comprises a first titanium silicalite with a titanium content of from 1.0 to 3.0 % by weight, calculated as TiO₂, and a second titanium silicalite with a titanium content of from 3.0 to 5.0 % by weight, calculated as TiO₂. The titanium content of the second titanium silicalite is at least 0.1 % by weight, calculated as TiO₂, higher than the titanium content of the first titanium silicalite and preferably at least 0.5 % by weight higher than the titanium content of the first titanium silicalite. The titanium content of a titanium silicalite can be determined by ICP-OES (inductively coupled plasma optical emission spectrometry). Titanium silicalites having different titanium contents as required by the invention can be synthesized with known methods for synthesizing titanium silicalites by varying the weight ratio of the silicon containing precursor compound and the titanium containing precursor compound used as a starting material in the titanium silicalite synthesis. Suitable methods for preparing titanium silicalite with a titanium content as required by the invention are known from the prior art, for example from US 4,410,501, EP 838 431, WO 01/64581 and WO 01/64582. The first titanium silicalite and the second titanium silicalite may have the same crystal structure or they may have different crystal structure. Preferably, both the first titanium silicalite and the second titanium silicalite have an MFI crystal structure. The epoxidation catalyst preferably comprises the first titanium silicalite and the second titanium silicalite in a weight ratio of from 20 : 1 to 1 : 20, more preferably from 4 : 1 to 1 : 4.

The use of an epoxidation catalyst comprising two titanium silicalites differing in their titanium content surprisingly provides better propene oxide yields compared to an epoxidation catalyst comprising the same amount of titanium in a single titanium silicalite and at the same time reduces hydrogen peroxide decomposition at the catalyst to molecular oxygen. The use of an epoxidation catalyst comprising a mixture of two titanium silicalites also surprisingly provides better propene oxide yields compared to the spatially separated use of the same titanium silicalites.

The epoxidation catalyst used in the process of the invention may comprise further titanium zeolites in addition to the first titanium silicalite and the second titanium silicalite, differing from them either by structure or by titanium content.

The epoxidation catalyst used in the process of the invention may be a powder catalyst or a shaped catalyst, with shaped catalysts being preferred. Suitable shaped catalysts may be prepared by any shaping method known to be useful for shaping a titanium silicalite, such as spray drying, fluidized bed spray agglomeration, mixing agglomeration or extrusion. Shaping may be carried out with addition of one or more binders or using all or a part of the liquid from a titanium silicalite synthesis step as a binder precursor. Compounds improving porosity or hardness of the shaped catalyst may be added in the shaping method. Suitable methods for preparing shaped catalysts comprising titanium silicalite are known from the prior art e.g. from EP 0 893 158 A1, WO 01/72419 and WO 01/72420.

In a first preferred embodiment, the epoxidation catalyst used in the process of the invention is a shaped catalyst comprising shaped bodies which contain both the first titanium silicalite and the second titanium silicalite. The shaped bodies then preferably all contain the first titanium silicalite and the second titanium silicalite in the same weight ratios. Shaped catalysts for use in this embodiment can be prepared be mixing the first titanium silicalite and the second titanium silicalite before shaping the catalyst.

In a second preferred embodiment, the epoxidation catalyst used in the process of the invention is a shaped catalyst comprising a mixture of two types of shaped bodies, the first type of shaped bodies containing only the first titanium silicalite and the second type of shaped bodies containing only the second titanium silicalite. The first type of shaped bodies and the second type of shaped bodies preferably have the same shape in order to prevent segregation of the two types of shaped bodies during handling or use of the shaped catalyst.

Shaped catalysts for use in the process of the invention are preferably prepared by an extrusion process and preferably comprise shaped bodies that are extrudates, preferably extrudates having a cylindrical shape with a diameter of from 1 to 5 mm and a length of from 1 to 7 mm.

The epoxidation of propene is preferably carried out in the presence of a solvent. Suitable are all solvents which are not oxidized or are oxidized to only a small extent by hydrogen peroxide under the reaction conditions chosen and which dissolve in water in an amount of more than 10 % by weight. Solvents which are completely miscible with water are preferred. Suitable solvents are alcohols, such as methanol, ethanol or tert-butanol; glycols, such as ethylene glycol, 1,2-propanediol or 1,3-propanediol; cyclic ethers, such as tetrahydrofuran, dioxane or propylene oxide; glycol ethers, such as ethylene glycol monomethyl ether, ethylene glycol monoethyl ether, ethylene glycol monobutyl ether or the propylene glycol monomethyl ethers; ketones, such as acetone or 2-butanone; and nitriles, such as acetonitrile and proprionitrile. Preferably, the solvent is a methanol solvent. The methanol solvent can be a technical grade methanol, a methanol solvent stream recovered in the work-up of the epoxidation reaction mixture or a mixture of both. The methanol solvent may comprise other solvents than methanol in minor amounts, such as ethanol, with the amount of such other solvents preferably being less than 2 % by weight. The methanol solvent may further comprise water. The solvent is preferably used in the epoxidation in a weight ratio of 0.5 to 20 relative to the combined weight of water and hydrogen peroxide.

When a shaped epoxidation catalyst is used with a methanol solvent, the fresh epoxidation catalyst or a dry regenerated epoxidation catalyst is preferably conditioned with methanol before start-up of the reaction. The catalyst may be conditioned by contacting it with methanol vapor or a gas stream comprising methanol vapor. Preferably, the catalyst is conditioned by contacting it with a first conditioning liquid comprising more than 60 % by weight water and less than 40 % by weight methanol to provide a conditioned catalyst and optionally further contacting it with at least one further conditioning liquid having a methanol content higher than the methanol content of the first conditioning liquid, where at least one of the conditioning liquids comprises water and from 25 to 45 % by weight methanol. The combined amount of water and methanol in the conditioning liquids is preferably at least 95 % by weight. The contacting with the one or more conditioning liquids is preferably carried out as described in international patent application PCT/EP2015/066814. Conditioning a dry shaped epoxidation catalyst as described above prevents breaking of the shaped catalyst upon contact with the liquid methanol solvent during start-up of the reaction.

The epoxidation of propene is preferably carried out at a pressure that is higher than the vapor pressure of propene at the reaction temperature in order to maintain the olefin dissolved in the solvent or present as a separate liquid phase. The pressure is preferably from 1.9 to 5.0 MPa, more preferably 2.1 to 3.6 MPa and most preferably 2.4 to 2.8 MPa. Propene is preferably used in an excess sufficient to maintain an additional liquid phase rich in propene throughout the epoxidation reaction. Using an excess of propene at a high pressure provides high reaction rate and hydrogen peroxide conversion and at the same time high selectivity for propene oxide.

The epoxidation of propene is preferably carried out at a temperature of 20 to 80°C, more preferably of 25 to 60°C.

The epoxidation of propene is preferably carried out in a fixed bed reactor by passing a mixture comprising propene and hydrogen peroxide through a fixed bed containing a shaped catalyst as described above. The mixture preferably comprises a solvent, more preferably a methanol solvent as described above. Most preferably, a mixture comprising propene in a molar excess to hydrogen peroxide and a methanol solvent is passed through the fixed bed at a temperature of from 20 to 80°C and a pressure of from 1.9 to 5.0 MPa. The fixed bed reactor is preferably equipped with cooling means and cooled with a liquid cooling medium. Preferably, a tube bundle reactor comprising a multitude of parallel reaction tubes and a cooling jacket enclosing the reaction tubes is used. The tube bundle reactor preferably comprises from 5000 to 20000 parallel reaction tubes, more preferably from 7500 to 15000 parallel reaction tubes. The reaction tubes preferably have a circular cross section with an internal diameter of preferably from 2 to 5 cm, more preferably from 2.5 to 4 cm. Preferably all reaction tubes of the tube bundle reactor have the same internal diameter. The reaction tubes preferably have a length of from 5 to 18 m, more preferably from 10 to 15 m. The catalyst fixed bed preferably extends over more than 70 % of the length of the reaction tubes, more preferably over 90 to 98 % of the length of the reaction tubes. Preferably, the temperature distribution along the length of the catalyst fixed bed is adjusted to keep the reaction temperature along 70 to 98 %, preferably along 80 to 95 %, of the length of the catalyst fixed bed within a range of less than 5 °C, preferably within a range of from 0.5 to 3 °C. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The epoxidation reaction is most preferably carried out with a catalyst fixed bed maintained in a trickle bed state at a pressure close to the vapor pressure of propene at the reaction temperature, using a methanol solvent and an excess of propene that provides a reaction mixture comprising two liquid phases, a methanol rich phase and a propene rich liquid phase. Two or more fixed bed reactors may be operated in parallel or in series in order to be able to operate the epoxidation process continuously when regenerating the epoxidation catalyst.

In a preferred embodiment, ammonia is added to the reaction mixture of in which propene is reacted with hydrogen peroxide with a weight ratio of ammonia to the initial amount of hydrogen peroxide of from 0.0001 to 0.003 in order to reduce by-product formation. When the epoxidation is carried out continuously, the ammonia is preferably added to a feed stream to the reaction, preferably to a solvent stream.

When the epoxidation is carried out continuously, the epoxidation catalyst will gradually lose catalytic activity during the course of the reaction. Preferably, the reaction temperature is increased during the course of the reaction in order to maintain sufficient conversion of hydrogen peroxide despite decreasing catalyst activity.

When, due to catalyst deactivation, the conversion of hydrogen peroxide falls below the desired level or the rise in reaction temperature necessary for maintaining the desired conversion of hydrogen peroxide leads to an undesired level of by-product formation, a continuous epoxidation is preferably interrupted to replace or regenerate the epoxidation catalyst. A shaped catalyst arranged in tubes of a tube bundle reactor is preferably regenerated within the reaction tubes. Regeneration within the reaction tubes can be achieved by methods known from the prior art, such as passing a gas stream at a temperature of from 200 to 600 °C through the catalyst fixed bed, passing a solvent stream through the catalyst bed or passing a solution of hydrogen peroxide through the catalyst bed in the absence of propene.

The epoxidation catalyst is preferably regenerated by passing a methanol solvent through a catalyst fixed bed of shaped catalyst at a temperature of from 100 to 200°C for a period of 0.5 to 48 hours, more preferably 20 to 36 hours and most preferably 20 to 24 hours. The methanol solvent used for regenerating the catalyst preferably comprises more than 90 % methanol and less than 10 % water and more preferably more than 96 wt.-% methanol and less than 4 % water. The methanol solvent is preferably a technical grade methanol, a solvent stream recovered in the work-up of the epoxidation reaction mixture or a mixture of both. The methanol solvent is preferably passed through the catalyst fixed bed in down flow mode and most preferably the flow rate is adjusted to maintain a trickle flow in the catalyst fixed bed. Regeneration may be performed at a constant temperature or using a temperature program. Passing the methanol solvent through the catalyst fixed bed is preferably started at the reaction temperature of the epoxidation reaction. The temperature is then raised to at least 100°C and maintained at a temperature of at least 100°C for the time necessary to carry out regeneration. Thereafter, the temperature is lowered back to the temperature used for epoxidation. Finally, the methanol flow is stopped or the epoxidation is recommenced by starting to feed propene and hydrogen peroxide to the fixed bed reactor. In such a temperature program, raising and lowering of the temperature is preferably performed at a rate of from 5 to 30 °C/h. During regeneration the pressure is adjusted to maintain the major part of the methanol solvent in the liquid state. The necessary pressure may be attained as the autogenous vapor pressure by evaporating part of the methanol solvent or by supplying an inert gas such as nitrogen. At least a part of the solvent that is passed through the catalyst fixed bed may be reused for regenerating the catalyst without prior purification. Preferably, the methanol solvent is passed through the catalyst fixed bed without reuse for a period of from 2 % to 70 % of the time used for regeneration and thereafter all the methanol solvent that is passed through the catalyst fixed bed is returned to the regeneration, creating a closed loop for washing the catalyst with a methanol solvent for the remainder of regeneration time. This reduces the amount of methanol needed for regenerating the catalyst.

The propene oxide formed by the epoxidation reaction can be separated from the epoxidation reaction mixture by methods known from the prior art, such as by distillation or extractive distillation. When a methanol solvent is used, propene oxide is preferably separated from the epoxidation reaction mixture by distillation after a pressure release stage which removes most of the non-reacted propene. The distillation is preferably carried out in at least two columns, operating the first column to provide a crude propene oxide overhead product containing from 20 to 60 % of the methanol contained in the epoxidation reaction mixture and further purifying the overhead product by at least one additional distillation. The overhead product is preferably further purified by distilling off remaining propene and propane, followed by extractive distillation, most preferably using the extractive distillation method of WO 2004/048355 for additional removal of carbonyl compounds.

When the epoxidation of propene is carried out with a methanol solvent, the reaction mixture is preferably subjected to a pressure reduction and propene vapor formed by the pressure reduction is recompressed and cooled to recover propene by condensation. The compressed propene vapor is preferably fed to a propene distillation column and separated into an overhead product comprising non-reacted propene and a bottoms product containing compounds having a boiling point higher than propene, such as propene oxide and methanol solvent. The overhead product comprising non-reacted propene can be recycled to the epoxidation reaction. The bottoms product can be combined with the liquid mixture remaining after the pressure reduction. The liquid mixture remaining after the pressure reduction is preferably separated by distillation in a pre-separation column to provide an overhead product comprising propene oxide, methanol and residual propene and a bottoms product comprising methanol, water and non-reacted hydrogen peroxide. The pre-separation column is preferably operated to provide an overhead product comprising from 20 to 60 % of the methanol contained in the liquid phase of the last pressure reduction step. The pre-separation column preferably has from 5 to 20 theoretical separation stages in the stripping section and less than 3 theoretical separation stages in a rectifying section and is most preferably operated without reflux and without a rectifying section to minimize the residence time of propene oxide in the pre-separation column. The pre-separation column is preferably operated at a pressure of from 0.16 to 0.3 MPa. Propene oxide and methanol are condensed from the overhead product of the pre-separation column and propene is preferably stripped from the resulting condensate in a propene stripping column which provides a bottom stream comprising propene oxide and methanol which is essentially free of propene. Propene oxide is preferably separated from the bottoms stream of the propene stripping column in an extractive distillation using water as the extraction solvent. The extractive distillation is preferably operated with additional feeding of a reactive compound containing an unsubstituted NH₂ group and capable of reacting with acetaldehyde during the extractive distillation, as described in WO 2004/048335. Extractive distillation with a reactive compound provides a high purity propene oxide containing less than 50 ppm of carbonyl compounds.

Methanol can be recovered from the bottoms product of the pre-separation column by distillation. Preferably, the bottoms product of the pre-separation column is subjected to a catalytic hydrogenation with hydrogen to remove non-reacted hydrogen peroxide, as described in WO 03/093255, before methanol is separated by distillation. Such catalytic hydrogenation reduces the amount of carbonyl compounds and acetals in the methanol separated by distillation, which is advantageous when the methanol is recycled to the epoxidation reaction. The bottoms product of the extractive distillation is preferably combined with the bottoms product of the pre-separation column, preferably before subjecting it to hydrogenation, in order to recover methanol. When hydrazine is used as the reactive compound in the extractive distillation, passing the bottoms product of the extractive distillation to the catalytic hydrogenation will convert non-reacted hydrazine and hydrazones formed from carbonyl compounds to ammonia and amines. The recovered methanol can be recycled as solvent to the epoxidation reaction. Preferably, the recovered methanol or the bottoms product of the pre-separation column, optionally combined with bottoms product of the extractive distillation and preferably after a catalytic hydrogenation, is treated to remove organic nitrogen compounds as described in WO 2004/048354, more preferably by subjecting it to an acid treatment. Most preferably, the recovered methanol is passed over a cation exchanger in the hydrogen form before it is recycled to the epoxidation reaction. Removal of organic nitrogen compounds, in particular amines, avoids deactivation of the titanium silicalite catalyst upon recycling of methanol.

### Examples

Titanium silicalite powders with titanium contents of 2.9, 3.6 and 4.3 % by weight, calculated as TiO₂, were prepared from silicon-titanium mixed oxides with titanium contents of 3.1, 3.9 and 4.7 % by weight, calculated as TiO₂, using the method described in example 1 of EP 0 814 058.

Extrudates were prepared by mixing titanium silicalite powder, a silica binder, water and a plasticizer, kneading, extruding, drying at 120 °C and calcining at 550 °C.

Epoxidation of propene was carried out with two fixed bed reactors, each containing 9 g catalyst in the form of extrudates, arranged in series and operated in up-flow. A first feed stream of 23 g/h methanol, 4 g/h hydrogen peroxide and 3 g/h water and a second feed stream of 20 g/h propene were both fed to the first reactor. Reaction pressure was maintained at 25 bar by a pressure retention valve after the second reactor. The reaction mixture leaving the second fixed bed reactor was depressurized to ambient pressure. The resulting gas phase was analyzed for propene, propene oxide and oxygen and the resulting liquid phase was analyzed for propene oxide and hydrogen peroxide. Propene oxide yields were calculated on the hydrogen peroxide charged with the feed stream.

### Example 1 (comparative)

The epoxidation was carried out using extrudates comprising a single titanium silicalite with a titanium content of 2.9 % by weight, calculated as TiO₂, in both fixed bed reactors. Table 1 gives the propene oxide yield and the fraction of hydrogen peroxide decomposed to oxygen after operating the reaction for 480 h.

### Example 2 (comparative)

Example 1 was repeated using extrudates comprising a single titanium silicalite with a titanium content of 3.6 % by weight, calculated as TiO₂, in both fixed bed reactors. Table 1 gives the propene oxide yield and the fraction of hydrogen peroxide decomposed to oxygen after operating the reaction for 480 h.

### Example 3 (comparative)

Example 1 was repeated using extrudates comprising a single titanium silicalite with a titanium content of 4.3 % by weight, calculated as TiO₂, in both fixed bed reactors. Table 1 gives the propene oxide yield and the fraction of hydrogen peroxide decomposed to oxygen after operating the reaction for 480 h.

### Example 4 (comparative)

Example 1 was repeated with the extrudates of example 1 in the first fixed bed reactor and the extrudates of example 3 in the second fixed bed reactor. Table 1 gives the propene oxide yield and the fraction of hydrogen peroxide decomposed to oxygen after operating the reaction for 480 h.

### Example 5

Example 1 was repeated with a mixture of equal amounts of the extrudates of example 1 and the extrudates of example 3 in both fixed bed reactors. Table 1 gives the propene oxide yield and the fraction of hydrogen peroxide decomposed to oxygen after operating the reaction for 480 h.

### Example 6

Example 1 was repeated with extrudates, where each extrudate comprised equal amounts of the titanium silicalite with a titanium content of 2.9 % by weight used in example 1 and of the titanium silicalite with a titanium content of 4.3 % by weight used in example 3, in both fixed bed reactors. Table 1 gives the propene oxide yield and the fraction of hydrogen peroxide decomposed to oxygen after operating the reaction for 480 h.

**Table 1**

| Example | Propene oxide yield in % | Fraction of H₂O₂ decomposed to O₂ in % |
|---|---|---|
| 1 | 63 | 3.8 |
| 2 | 71 | 5.3 |
| 3 | 73 | 5.7 |
| 4 | 71 | 4.0 |
| 5 | 71 | 4.3 |
| 6 | 73 | 4.1 |

The examples demonstrate that the claimed process, which uses an epoxidation catalyst comprising two titanium silicalites with different titanium contents, provides better propene oxide yields at reduced hydrogen peroxide decomposition compared to using an epoxidation catalyst comprising a single titanium silicalite with a comparable titanium content and provides better propene oxide yields compared to using two spatially separated titanium silicalites.

## Claims

1. A process for the epoxidation of propene with hydrogen peroxide in the presence of an epoxidation catalyst, wherein the epoxidation catalyst comprises a first titanium silicalite with a titanium content of from 1.0 to 3.0 % by weight, calculated as TiO₂, and a second titanium silicalite with a titanium content of from 3.0 to 5.0 % by weight, calculated as TiO₂, the titanium content of the second titanium silicalite being at least 0.1 % by weight, calculated as TiO₂, higher than the titanium content of the first titanium silicalite.

2. The process of claim 1, wherein the titanium content of the second titanium silicalite is at least 0.5 % by weight, calculated as TiO₂, higher than the titanium content of the first titanium silicalite.

3. The process of claim 1, wherein the epoxidation catalyst comprises the first titanium silicalite and the second titanium silicalite in a weight ratio of from 20 : 1 to 1 : 20, preferably from 4 : 1 to 1 : 4.

4. The process of any one of claims 1 to 3, wherein the epoxidation catalyst is a shaped catalyst comprising shaped bodies which contain both the first titanium silicalite and the second titanium silicalite.

5. The process of any one of claims 1 to 3, wherein the epoxidation catalyst is a shaped catalyst comprising a mixture of two types of shaped bodies, the first type of shaped bodies containing only the first titanium silicalite and the second type of shaped bodies containing only the second titanium silicalite.

6. The process of claim 5, wherein the first type of shaped bodies and the second type of shaped bodies have the same shape.

7. The process of any one of claims 4 to 6, wherein the shaped bodies are extrudates.

8. The process of claim 7, wherein the extrudates have a cylindrical shape with a diameter of from 1 to 5 mm and a length of from 1 to 7 mm.

9. The process of any one of claims 4 to 8, wherein a mixture comprising propene and hydrogen peroxide is continuously passed through a fixed bed containing the shaped catalyst.

10. The process of claim 9, wherein the mixture comprises propene in a molar excess to hydrogen peroxide and methanol as solvent and is passed through the fixed bed at a temperature of from 20 to 80°C and a pressure of from 1.9 to 5.0 MPa.
